# EUROPEAN PATENT APPLICATION

(11) **EP 2 305 277 A1**
(43) Date of publication of application: **06.04.2011**
(21) Application number: 09170746.3
(22) Date of filing: 18.09.2009
(51) Int. Cl.: A61K 35/14, C12N 5/0784, A61K 39/00

(54) **Use of tolerogenic dendritic cells in treatment and prevention of atherosclerosis**

(71) Applicant: Forskarpatent I Syd AB, 223 70 Lund (SE)
(72) Inventor: Hansson, Göran, 112 39, STOCKHOLM (SE); Hermansson, Andreas, 172 78, SUNDBYBERG (SE)
(74) Representative: Stenbäck, Maria Elisabeth

(57) **Abstract**

The present invention relates to the use of tolerogenic dendritic cells in treatment and/or prevention of atherosclerosis. The cells have preferably been pretreated with at least one cytokine selected from the TGF-beta superfamily, such as TGF-beta-2, and/or with interleukin-10. Preferably, the cells have also been loaded with an autoantigen of atherosclerosis, such as the LDL protein, ApoB100 or a peptide thereof.

## Description

### Field of the invention

The present invention relates to use of tolerogenic dendritic cells in treatment and prevention of atherosclerosis.

### Background

Atherosclerosis is a chronic inflammatory disease characterized by a massive intimal deposit of cholesterol derived from low density lipoprotein (LDL), and by an inflammatory response against accumulated LDL.

More precisely, atherosclerosis is a chronic disease that causes a thickening of the innermost layer (the intima) of large and medium-sized arteries. It decreases blood flow and may cause thrombosis, ischemia and tissue destruction in organs supplied by the affected vessel. Atherosclerosis is the major cause of cardiovascular disease including myocardial infarction, stroke and peripheral artery disease. It is the major cause of death in the Western world and is predicted to become the leading cause of death in the entire world within two decades.

Research during the last 20 years has shown that atherosclerosis is an inflammatory disease, which develops at sites of cholesterol accumulation in the artery wall.

The importance of adaptive immunity for atherosclerosis is supported by the finding of antibodies and T cells reactive to components of LDL, and further underlined by the finding that severe combined immunodeficiency (SCID) leads to reduced atherosclerosis in hypercholesterolemicexperimental animals.

The LDL particle consists of several different molecules including triglycerides, cholesterol esters, phospholipids, and a large protein, apolipoprotein B100 (ApoB100).

The mechanisms of atherosclerosis have been described earlier, for example by G. K. Hansson in N. Engl. J. Med. 2005;352:1685-95, which is incorporated herein by reference.

### Summary of the invention

One aspect of the present invention relates to tolerogenic dendritic cells for use in treatment and/or prevention of atherosclerosis.

Another aspect of the present invention relates to tolerogenic dendritic cells which have been pretreated with at least one cytokine selected from the transforming growth factor-beta (TGF-beta) superfamily, such as TGF-beta-2, and/or with interleukin-10 for use in treatment and/or prevention of atherosclerosis.

Another aspect of the present invention relates to tolerogenic dendritic cells which have been loaded with an autoantigen of atherosclerosis, such as ApoB100 or a peptide thereof, for use in treatment and/or prevention of atherosclerosis.

Another aspect of the invention is a method for treatment of atherosclerosis wherein tolerogenic dendritic cells as described above are administered to a patient in need thereof.

Another aspect of the invention is a pharmaceutical composition, intended for use in treatment of atherosclerosis, comprising tolerogenic dendritic cells as described above.

Some of the expressions and terms used in this application are explained below in order to avoid facilitate understanding of the invention.

The term "tolerogenic", as it is used herein, relates to a substance that can lead to immunological tolerance.

The term "antigen", as it is used herein, relates to any substance that, when introduced into the body can stimulate an immune response. An "autoantigen" is an antigen that despite being a normal tissue constituent is the target of a humoral or cell-mediated immune response.

The term "treatment" used herein relates to treatment in order to cure or alleviate a disease or a condition. The treatment may either be performed in an acute or in a chronic way.

The term "patient", as it is used herein, relates to any human in need of treatment according to the invention.

The term "prevention" used herein relates to treatment in order to prevent the development of a disease or a condition. The preventive treatment is normally used on individuals who have not yet shown any clinical signs of atherosclerosis.

The term "therapeutically effective amount" relates to an amount that will lead to the desired therapeutic effect, i.e. to an effect on atherosclerosis.

The terms "comprises" and "comprising" used herein means including but not limited to.

### Detailed description to the inventors

In the work leading to the present invention the inventors tested treatment of atherosclerosis by dampening the inflammatory response to LDL by injecting mice intravenously with dendritic cells (DC) that had been pulsed with the protein component of LDL apolipoprotein B100 (ApoB100) in combination with the immunosuppressive cytokines IL-10 and/or TGFbeta2. Spleen cells from such mice showed diminished proliferative responses to ApoB100 as compared to cells from mice injected with DC not treated with immunosuppressive cytokines. The dampened immunity was also reflected in the cytokine pattern from the spleen cells with significantly decreased levels of IFN-gamma, IL-5, IL-6, IL-12 and TNF-alpha. To test the effect of tolerogenic DC on atherosclerosis, disease prone huApoB100tg x Ldlr-/- mice were injected with the ApoB100-pulsed tolerogenic dendritic cells. This led to a very significant reduction of atherosclerotic lesions in the aorta, as compared with untouched controls or those injected with DC treated with antigen or cytokine alone. It was thus found that tolerogenic DC pulsed with ApoB100 reduce the autoreactive response against LDL. This leads to novel possibilities for treatment and/or prevention of atherosclerosis.

As mentioned above, the invention thus relates to tolerogenic dendritic cells for use in treatment and/or prevention of atherosclerosis.

The dendritic cells (DC) may be of any origin, although human origin is preferred. Said tolerogenic dendritic cells may be obtained by treatment of dendritic cells (DC) with at least one cytokine selected from the TGF-beta superfamily and/or with interleukin-10.

It is possible to use only one cytokine from the TGF-beta superfamily or interleukin-10. It is also possible to use a combination of one or more cytokine from the TGF-beta superfamily and/or interleukin-1 0.

Cytokines belonging to the TGF-beta superfamily that may be used according to the present invention are TGF-beta 1, TGF-beta 2, TGF-beta 3, TGF-beta 4, TGF-beta 5, inhibins, activins, Mullerian inhibitory substance, bone morphogenetic proteins (BMPs), Growth and Differentiation Factors of the GDF family, and the GDNF family. They include AMH; ARTN; BMP10; BMP15; BMP2; BMP3; BMP4; BMP5; BMP6; BMP7; BMP8A; BMP8B; BMP10; BMP15; GDF1; GDF10; GDF11; GDF15; GDF2; GDF3; GDF3A; GDF5; GDF6; GDF7; GDF8; GDF9; GDNF; INHA; INHBA; INHBB; INHBC; INHBE; LEFTY1; LEFTY2; MSTN; NODAL; NRTN; PSPN; TGFB1; TGFB2; TGFB3.

According to one aspect, the cytokine selected from the TGF-beta superfamily used is TGF-beta-2.

This treatment or pretreatment with at least one cytokine selected from the TGF-beta superfamily and/or with interleukin-10 is made by incubating the dendritic cells in the presence the cytokine from the TGF-beta superfamily and/or with interleukin-10.

DC are isolated from bone marrow or blood by standard protocols and stimulated to differentiate by cytokine treatment in cell culture. Common methods to achieve such DC were described by by Y.I. Son et al., A novel bulk-culture method for generating mature dendritic cells from mouse bone marrow cells. J lmmunol Methods 2002;262:145-157, and by K. Inaba et al. in Curr. Protoc. Immunol.86:3.7.1-3.7.19, 2009 (John Wiley & Sons, Inc). The DC obtained in this way are incubated with IL-10 and/or a TGF-beta family member. Recombinant IL-10 and TGF-beta2 proteins are commonly used for this purpose but isolated natural proteins may also be used. Conditions suitable for incubation with cytokines and antigen are described by YY. Lan et al. in "Alternatively activated" dendritic cells preferentially secrete IL-10, expand Foxp3+CD4+ T cells, and induce long-term organ allograft survival in combination with CTLA4-lg. J. lmmunol. 2006;177:5868-5877.

According to another aspect, the tolerogenic dendritic cells are pretreated with interleukin-10.

The tolerogenic dendritic cells according to the invention are preferably loaded with an autoantigen of atherosclerosis.

That the tolerogenic dendritic cells are loaded with an autoantigen of atherosclerosis means that the tolerogenic dendritic cells present such an autoantigen. This may be achieved by incubating the cells in the presence of the autoantigen. In a preferred approach, purified DC are incubated with optimal concentrations of recombinant IL-10 or TGF-beta2 together with autoantigen in serum-free cell culture medium for 18 hours. After 4 hours of incubation, lipopolysaccharide is added to induce DC maturation. After 18 hrs, DC are washed in medium and either injected or kept on ice until time of injection into the patient.

The autoantigen of atherosclerosis used in the present invention may be ApoB100. Alternatively, it may be an LDL particle containing apoB100, or a fragment or peptide component of ApoB100. The fragment or peptide component shall then have the essentially the same antigenic properties as ApoB100. Examples of such peptides are described in Fredrikson GN et al, Identification of immune responses against aldehyde-modified peptide sequences in apoB associated with cardiovascular disease. Arterioscler Thromb Vasc Biol 2003;23:872-8 and in Fredrikson GN et al., Inhibition of atherosclerosis in apoE-null mice by immunization with apoB-100 peptide sequences. Arterioscler Thromb Vasc Biol 2003;23:879-84. It may also be a combination of two or more of these autoantigens. In a preferred design, these autoantigens will be of human origin or will be recombinant or synthetic molecules with sequences identical to those occurring in humans. In an alternative approach, a molecule derived from another species will be used to induce the tolerogenic response.

According to the method for treatment of atherosclerosis according to the present invention, a therapeutically effective amount of the tolerogenic dendritic cells are administered to a patient in need of treatment and/or to an individual in need of prevention of atherosclerosis.

The pharmaceutical composition according to the invention, used according to the invention or produced according to the invention may also comprise other substances, such as an inert vehicle, or pharmaceutical acceptable adjuvants, carriers, preservatives etc., which are well known to persons skilled in the art.

The pharmaceutical composition may be formulated as a form suitable for intravenous injection, but also as, cutaneous, subcutaneous, nasal, peroral, intramuscular or intraperitoneal administration. In some cases, nasal or peroral administration may be preferred. Regardless of the route of administration, the pharmaceutical composition according to the invention is formulated into pharmaceutically acceptable dosage forms by conventional methods. The amount of the active ingredient will vary depending on the patient to be treated, due to factors such as age, sex, weight etc, and on the rout of administration as well as on other factors known to the skilled person.

The invention is further illustrated below in the following Examples.

### Brief description of the drawings

In the Examples, reference is made to the accompanying drawings on which:
Fig. 1 illustrates atherosclerotic lesion size in the proximal aorta. Lesions were measured as described (Nicoletti A et al, J Clin Invest 1998;102:910-918). Abbreviations are described in text. **) significantly different, P<0.01.
Fig. 2 illustrates T cell activation in response to ApoB100 in splenocytes of mice from the different treatment groups. Activation was measured as incorporation of 3H-thymidine into DNA and is shown as stimulation index (cpm of treated group - cpm for control / cpm for control: Fig. 2a and Fig. 2b), and also as raw data (cpm) (Fig. 2b).
Fig. 3 illustrated cytokine secretion in response to ApoB100 in splenocytes from mice of the different treatment groups. Interferon-gamma (Fig. 3a), interleukin-5 (Fig. 3b), interleukin-6 (Fig. 3c) and tumor necrosis factor-alpha (Fig. 3d) were measured in culture supernatants by ELISA.
Fig. 4 illustrates the effect of tolerogenic DC on antigen-specific activation of the ApoB100 specific T cell hybridoma, 48-5. Activation was measured as interleukin-2 secretion into the culture medium and by using ELISA.

### Examples

### Material and Methods

### Preparation of ApoB100

LDL (d = 1.019 - 1.063 g/mL) was isolated by ultracentrifugation from pooled plasma of healthy donors, as described (Havel R et al., J Clin Invest 34:1345-1353, 1955). After isolation, LDL was dialyzed extensively against PBS. One millimolar EDTA was added to an aliquot of LDL to generate unmodified LDL. ApoB100 was obtained as described (Wessel D and Flugge Ul, Anal Biochem 138:141-143, 1984), using minor modifications. Briefly, 0.4 ml methanol, 0.1 ml chloroform, and 0.3 ml water were added to 0.1 ml of LDL (1 mg/mL); the suspension was then vortexed and centrifuged at 9000 x g for 1 min. The upper phase was removed and 0.3 ml of methanol added to the lower phase and interphase with precipitated protein, which was mixed again and centrifuged at 9000 x g for 2 min to pellet the protein.

To obtain soluble and pure ApoB100, the protein pellet was resuspended in a minimum volume of 10% SDS (Bio-Rad Laboratories, Hercules, CA, USA) until it solubilized. These preparations first were filtered on a PD-10 column (GE Healthcare, Uppsala, Sweden) to remove excess SDS. They were then purified on a Superdex-200 size-exclusion column (0.5 mL/min, in Tris-HCl, pH 7.4). The first peak that contained ApoB100 was collected, and the extra peaks containing contaminant protein were discarded.

ApoB100 preparations were greater than 90% pure, as evaluated in a second injection into a Superdex-200 column (GE Healthcare, Uppsala, Sweden). Finally, protein concentration was determined by Bradford assay (Bio-Rad Laboratories, Hercules, CA, USA).

### Isolation and loading of dendritic cells

Dendritic cells (DC) were generated by a method adopted from Son et al. (J Immunol Methods. 2002;262:145-57). Femurs and tibias were dissected from mice and the ends of the bones were cut with scissors. The bone marrow was flushed from the bones with DMEM (Gibco) supplemented with 10% FCS. The collected bone marrow was meshed through a 100µm dispenser (Falcon) and centrifuged. The bone marrow cells were depleted of red blood cells by incubating with ACK-lysing buffer and cultured for 8 days in tissue culture dishes (100mmØ) (1x107cells/10ml/dish) in 37°C and 7,5% CO2. The culture medium used was DMEM supplemented with 10% FCS, 50U/ml penicillin, 50µg/ml streptomycin, 2mM L-glutamine, 1mM sodium pyruvate, 10ng/ml GM-CSF (Peprotech) and 10ng/ml IL-4 (Peprotech). Every 2-3 days 1/2 of the medium was replaced with fresh medium and cytokines. Purification of DC from the differentiated bone marrow cells was performed using a CD11 c magnetic cell sorting kit (Miltenyi Biotech) according to the manufacturers' instructions.

For cell transfer experiments we used a slightly modified version of previously published protocols (J. Immunol. 2006;177:5868-5877 and J. Immunol. 2004;172:1991-1995), briefly the purified DC were incubated at a density of approximately 1x10⁶ cells/ml in tissue culture dishes (60mmØ) with or without 100µg/ml ApoB100 and with either IL-10 (30ng/ml) or TGFβ2 (5ng/ml) in serum-free DMEM medium containing 1:100 BD ITS+ Premix (BD Biosciences, Franklin Lakes, NJ, USA), 1 mg/mL BSA (Sigma-Aldrich, St. Louis, MO, USA), 10 mmol/L HEPES (Gibco Invitrogen, Carlsbad, CA, USA), 1 mmol/L Na pyruvate (Gibco Invitrogen, Carlsbad, CA, USA), 1 mmol/L nonessential amino acids (Sigma-Aldrich, St. Louis, MO, USA), and 50 µg/mL gentamycin sulfate (Sigma-Aldrich, St. Louis, MO, USA), at 37°C in a humid 5% CO2 atmosphere. After 4 hours of culture LPS (0,1 ng) was added and the cells were further pulsed for another 14 hours. After pulsing, the cells were washed in DMEM and kept on ice until injected. For measuring cytokine release into the supernatant, the purified DC were pulsed in 96-well plates, 4x105 cells /well.

### Animal experiments and evaluation of disease

To study the effect of tolerogenic dendritic cells in disease development, the inventors injected 11-week-old male huB100tg x Ldlr-/- mice (Skalen et al., 2002, Nature 417:750-4) i.v. with 2.5x10⁵ DC pulsed with antigen and/or cytokines. The mice were fed a high-fat diet (0.15% cholesterol), starting 5 days after the immunization until sacrifice 10 weeks later with CO₂. In some experiments the mice were also immunized subcutaneously with 100ug of ApoB100 emulsified with CFA. All experiments were approved by the local ethics committee.

Blood from sacrificed mice was collected by cardiac puncture and vascular perfusion with sterile RNase-free PBS. Thoracic aortas and hearts were dissected and preserved for lesion analysis. One-third of the spleen was saved for cell experiments, one-third was snap-frozen for RNA isolation and one-third was frozen for cryostat sectioning. Inguinal and lumbar lymph nodes, liver and abdominal aortas also were snap-frozen and saved for RNA isolation.

### In vitro cell culture assays

Splenocytes from treated mice were isolated and resuspended. In 96-well plates, 5 x 10⁵ splenocytes were incubated with or without ApoB100 antigen in 200 µL of DMEM supplemented with 2.5% mouse serum, 50U/ml penicillin, 50µg/ml streptomycin, 2mM L-glutamine, 1mM sodium pyruvate for 72 hours at 37°C in a humid 5% CO₂ atmosphere. One microcurie ³H - thymidine (Sigma-Aldrich, St. Louis, MO, USA) was added after 60 h, and DNA replication was measured with a scintillation counter (Wallac, Turku, Finland). Results are expressed as stimulation index = ((s - c)/c, where s is the cpm of the sample with antigen and c is the cpm of the sample without antigen. Cytokines secreted in the cell culture supernatants after 72 hours were measured with a mouse IFN-gamma ELISA kit (Mabtech, Sweden) and with the cytometric bead array flex set kit (BD Biosciences, CA, USA) for IL-6, TNF-alpha, and IL-5.

In some experiments, 4x10⁵ antigen-pulsed and cytokine-treated DC were incubated with 1x10⁵ cells of the T cell hybridoma 48.5, which can recognize human ApoB100 in an I-Ab restricted manner and release IL-2 upon activation. This cytokine was measured with mouse IL-2 ELISA kit (R&D systems, USA).

### Statistical analysis

Values are expressed as mean ± standard error of the mean (SEM) unless otherwise indicated. The nonparametric Mann-Whitney U test was used for pairwise comparisons. Differences between groups were considered significant at P values below 0.05.

### Results

### Mice receiving IL-10- or TGFβ2-treated DC presenting ApoB100 show significantly reduced atherosclerosis

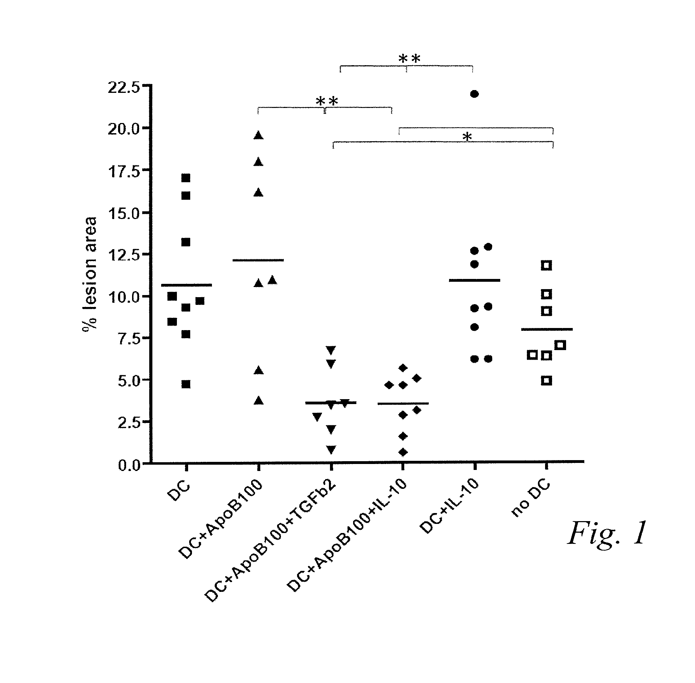

The inventors used huB100tg x Ldlr-/- mice as recipients of tolerogenic dendritic cells. These mice express full-length human ApoB100 in the liver and gut, and display humanized lipoprotein profiles. The huB100tg x Ldlr-/- model permits the use of human ApoB100 as antigen. The mice received one I.V. injection of DC that had been pulsed with either a) medium alone; b) ApoB100; c) ApoB100 + TGFβ2; d) ApoB100 + IL-10; or e) IL-10. Finally, f) one group of mice remained untreated. A massive decrease in lesion area of the descending thoracic aorta was evident and statistically significant for the groups receiving ApoB100 + TGFβ2 or ApoB100 + IL-10 as compared to those groups receiving only antigen or cytokines (Figure 1).

### In vivo transfer of IL-10- or TGFβ2-treated ApoB100-pulsed DC reduces splenocyte proliferation to ApoB100

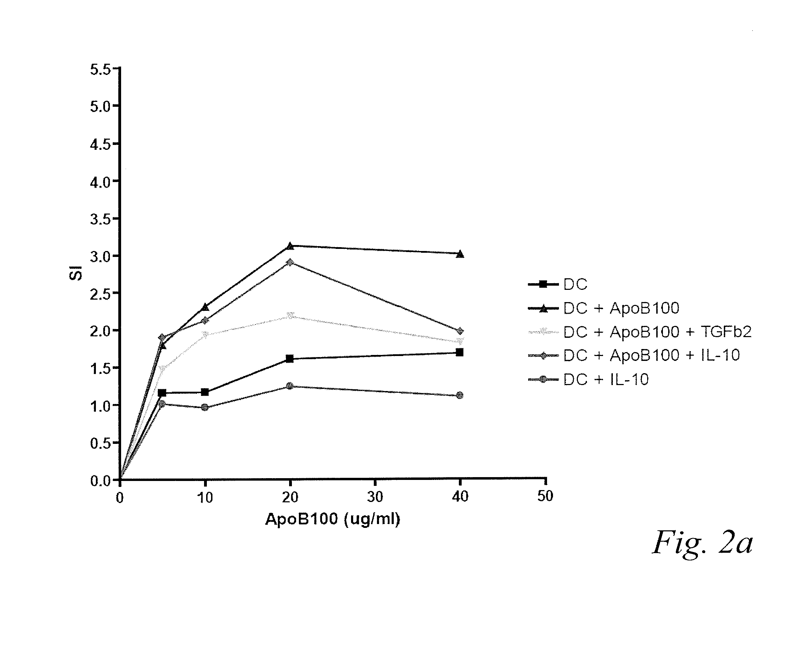

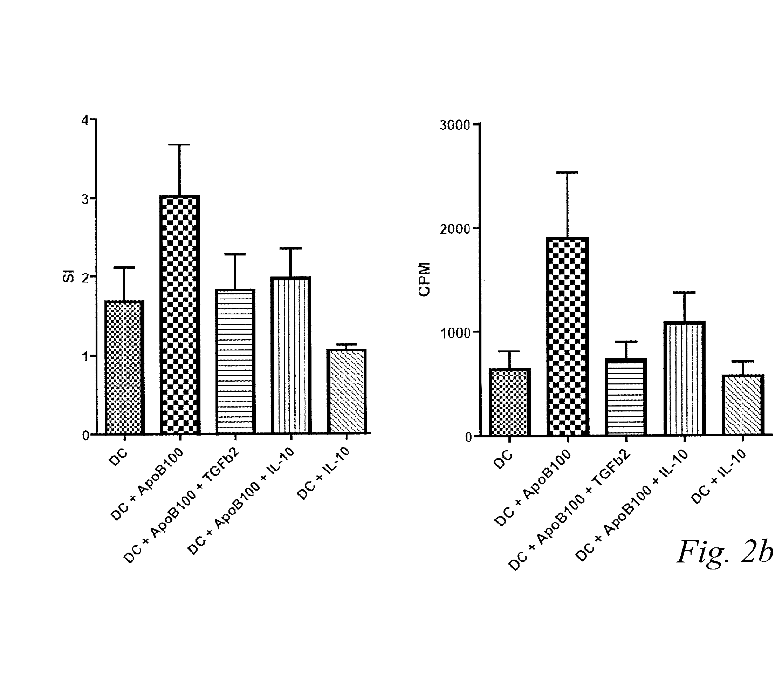

T cell populations are known to contain effector T cells reactive to LDL components (Stemme S et al, Proc Natl Acad Sci USA 1995; 92(9):3893-7; Zhou X et al, Arterioscl Thromb Vasc Biol 2006; 26(4):864-70). To test whether this response can be suppressed the inventors transferred DC pulsed with ApoB100 and treated with either TGFβ2 or IL-10 to mice (as above). One week later, these mice were immunized with 100ug of ApoB100 s.c. to boost the cellular response to the protein. After another week the in vitro proliferative response of splenocytes to ApoB100 was measured. Mice injected with DC pulsed with TGFβ2 or IL-10 and ApoB100 displayed a suppressed proliferative response to ApoB100 when re-stimulated in vitro (Figure 2). Therefore, DC treatment dampens the autoreactive immune response to ApoB100.

### In vivo transfer of IL-10- or TGFβ2-treated ApoB100-pulsed DC dampens pro-inflammatory cytokine responses

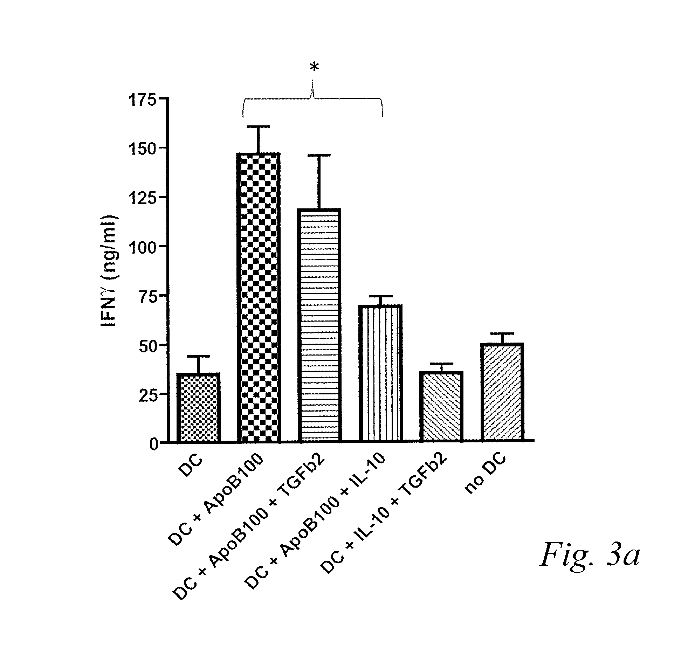

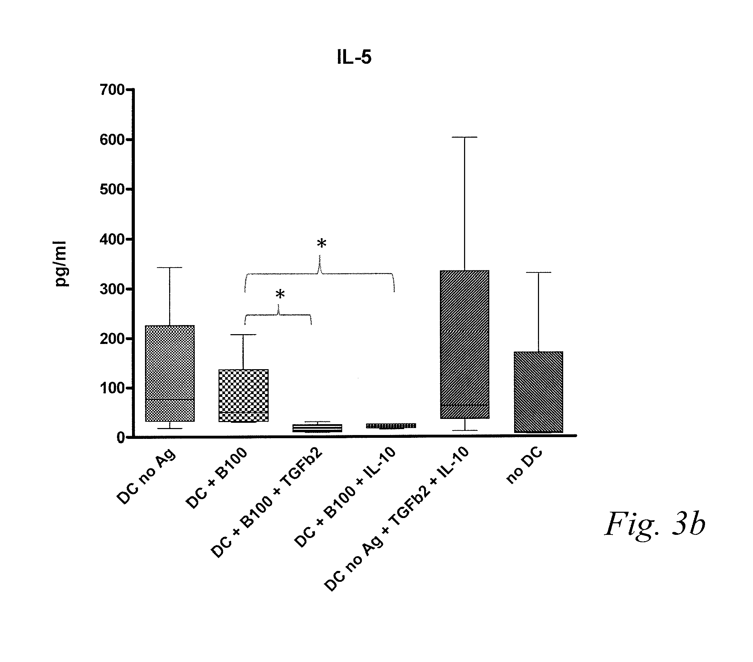

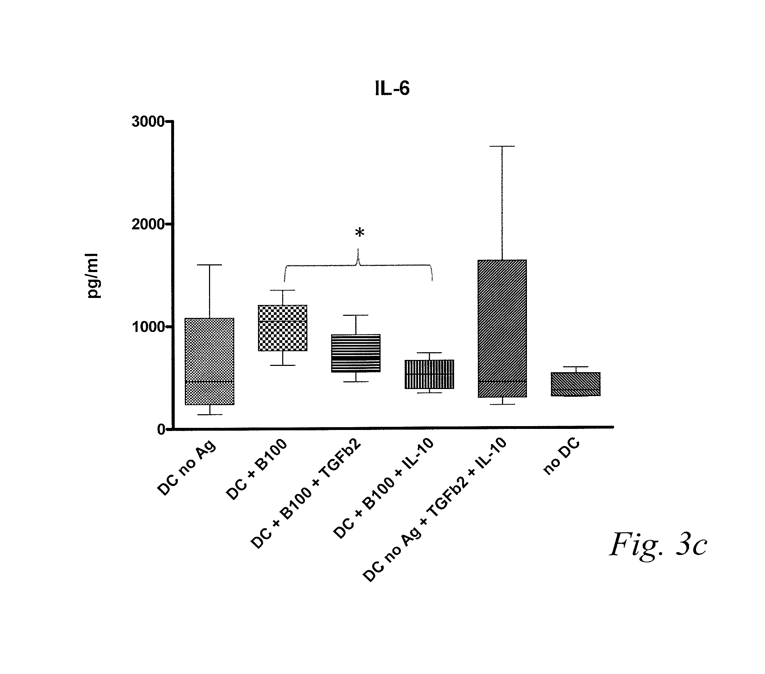

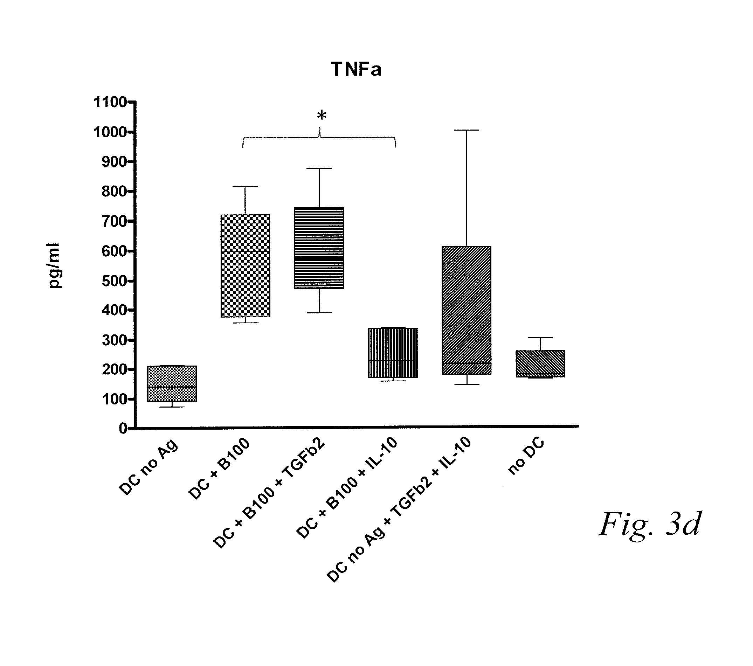

To further investigate the effect of tolerogenic DC transfer on the inflammatory properties of immune cells, supernatants of splenocyte cultures from the in vitro ApoB100 re-stimulation experiment (see above) were analyzed with regards to cytokines secreted. Concentrations of IFN-gamma, IL-5, IL-6 and TNF-alpha were significantly reduced in splenocyte cultures of mice injected with DC pulsed with ApoB100 and treated with IL-10, compared to ApoB100-pulse without IL-10 (Figure 3.). Splenocytes from mice injected with DC pulsed with ApoB100 and TGFβ2 secreted significantly less IL-5 in response to ApoB100, and showed a trend towards diminished production of IFN-gamma and IL-6 (Figure 3). This demonstrates that in vivo DC treatment reduces the autoimmune effector response against ApoB100.

### DC pulsed with ApoB100 and treated with IL-10 or TGFβ2 can suppress T cell response to ApoB100

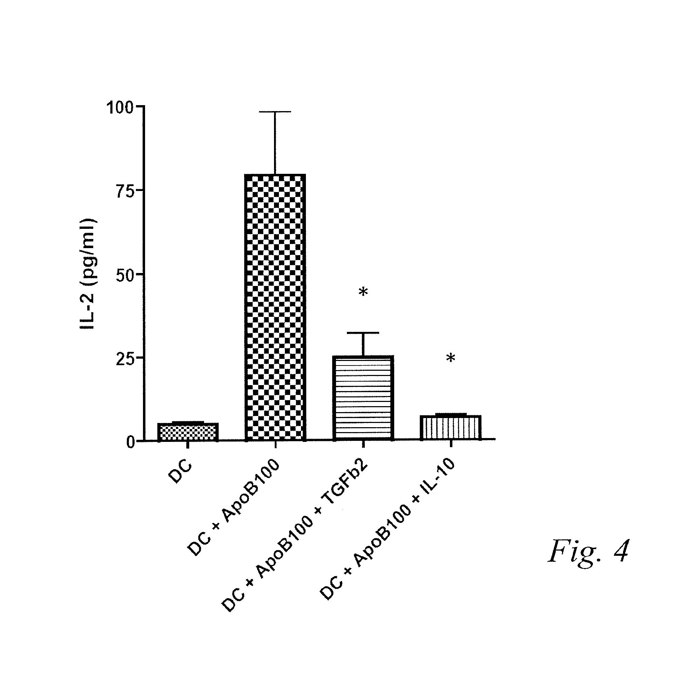

LDL reactive T cells are of a phenotype (CD4+, MHC class II restricted) known to recognize protein components (Stemme S 1995; Zhou X 2006, full references given above). The inventors hypothesized that by making the DC tolerogenic with IL-10 or TGFβ2 treatment in the context of ApoB100, the responding T cells would be less activated. When DC, pulsed with ApoB100 and IL-10 or TGFβ2, were incubated with the ApoB100-specific T cell hybridoma 48.5, they suppressed its activation, as reflected in a significantly reduced IL-2 production when compared to responses to DC pulsed with ApoB100 only (Figure 4). These data suggest that in vivo DC treatment dampens inflammation and disease by directly acting on the ApoB100 specific T cells.

## Claims

1. Tolerogenic dendritic cells for use in treatment and/or prevention of atherosclerosis.

2. Tolerogenic dendritic cells according to claim 1, wherein said tolerogenic dendritic cells have been pretreated with at least one cytokine selected from the TGF-beta superfamily and/or with interleukin-10.

3. Tolerogenic dendritic cells according to claim, which have been pretreated with TGF-beta-2.

4. Tolerogenic dendritic cells according to claim 2 or 3, which have been pretreated with interleukin-10.

5. Tolerogenic dendritic cells according to any one of the claims 1-4, which have been loaded with an autoantigen of atherosclerosis.

6. Tolerogenic dendritic cells according to claim 5, wherein said autoantigen is ApoB100.

7. Tolerogenic dendritic cells according to claim 5, wherein said autoantigen is a peptide component of ApoB100.

8. A pharmaceutical composition comprising tolerogenic dendritic cells.

9. A pharmaceutical composition according to claim 8, wherein said tolerogenic dendritic cells have been pretreated with at least one cytokine selected from the TGF-beta superfamily, such as TGF-beta-2, and/or with interleukin-10.

10. A pharmaceutical composition according to claim 8, wherein said tolerogenic dendritic cells have been loaded with an autoantigen of atherosclerosis, such as ApoB100, an ApoB100 containing lipoprotein such as LDL, or a peptide component of ApoB100.

11. A method for treatment and/or prevention of atherosclerosis wherein a therapeutically effective amount of tolerogenic dendritic cells are administered to a patient in need of treatment or to an individual in need of prevention.

12. The method of claim 11, wherein the tolerogenic dendritic cells have been pretreated with at least one cytokine selected from the TGF-beta superfamily and/or with interleukin-10.

13. The method of claim 12, wherein the tolerogenic dendritic cells have been pretreated with TGF-beta-2.

14. The method of claim 12 or 13, wherein the tolerogenic dendritic cells have been pretreated with interleukin-10.

15. The method of any one of the claims 11-14, wherein the tolerogenic dendritic cells have been loaded with an autoantigen of atherosclerosis.

16. The method of claim 15, wherein the autoantigen is ApoB100.

17. The method of claim 15, wherein the autoantigen is a peptide component of ApoB100.
